(19) Europäisches Patentamt
European Patent Office
Office européen des brevets

(11) EP 2 226 321 A2

(12) # EUROPEAN PATENT APPLICATION

(43) Date of publication:
**08.09.2010 Bulletin 2010/36**

(21) Application number: **10006276.9**

(22) Date of filing: **24.01.2006**

(51) Int Cl.:
*C07D 417/12* (2006.01) *A61K 31/427* (2006.01)
*A61P 3/10* (2006.01) *C07D 277/34* (2006.01)

(84) Designated Contracting States:
**AT BE BG CH CY CZ DE DK EE ES FI FR GB GR HU IE IS IT LI LT LU LV MC NL PL PT RO SE SI SK TR**

(30) Priority: **24.01.2005 JP 2005014930**

(62) Document number(s) of the earlier application(s) in accordance with Art. 76 EPC:
**06712274.7 / 1 894 929**

(71) Applicant: **Daiichi Sankyo Company, Limited**
**Tokyo 103-8426 (JP)**

(72) Inventors:
- **Nakamura, Yoshitaka**
**Hiratsuka-shi**
**Kanagawa 254-0014 (JP)**
- **Satoh, Chiharu**
**Hiratsuka-shi**
**Kanagawa 254-0014 (JP)**
- **Miyamoto, Hiroshi**
**Hiratsuka-shi**
**Kanagawa 254-0014 (JP)**
- **Kajino, Hisaki**
**Hiratsuka-shi**
**Kanagawa 254-0014 (JP)**

(74) Representative: **Fairbairn, Angus Chisholm**
**Marks & Clerk LLP**
**90 Long Acre**
**London**
**WC2E 9RA (GB)**

Remarks:
This application was filed on 17-06-2010 as a divisional application to the application mentioned under INID code 62.

(54) **Process for producing thiazolidinediones and intermediates thereof**

(57) There is provided a process for producing a thiazolidinedione derivative having excellent prophylactic and therapeutic effects on a disease caused by insulin resistance with a high purity; a process for producing a compound represented by the general formula (A) or a salt thereof, comprising converting 4-[(2,4-dioxothiazolidin-5-yl)methyl] phenoxyacetic acid represented by the following formula into a compound of the general formula (I), wherein X = a halogen atom; then reacting the compound with a compound represented by the general formula (II), wherein $R^1$, $R^2$, $R^3$ and $R^4$ = a hydrogen atom, a hydroxyl group, C1-C6 alkyl, C1-C6 alkoxy, benzyloxy, acetoxy, trifluoromethyl or a halogen atom, $R^5$ = C1-C6 alkyl, and $R^6$ = a protecting group for an amino group, to produce a compound represented by the general formula (III); and subsequently cyclizing the compound into the final product, and crystals of a compound represented by the general formula (A) or a salt thereof.

EP 2 226 321 A2

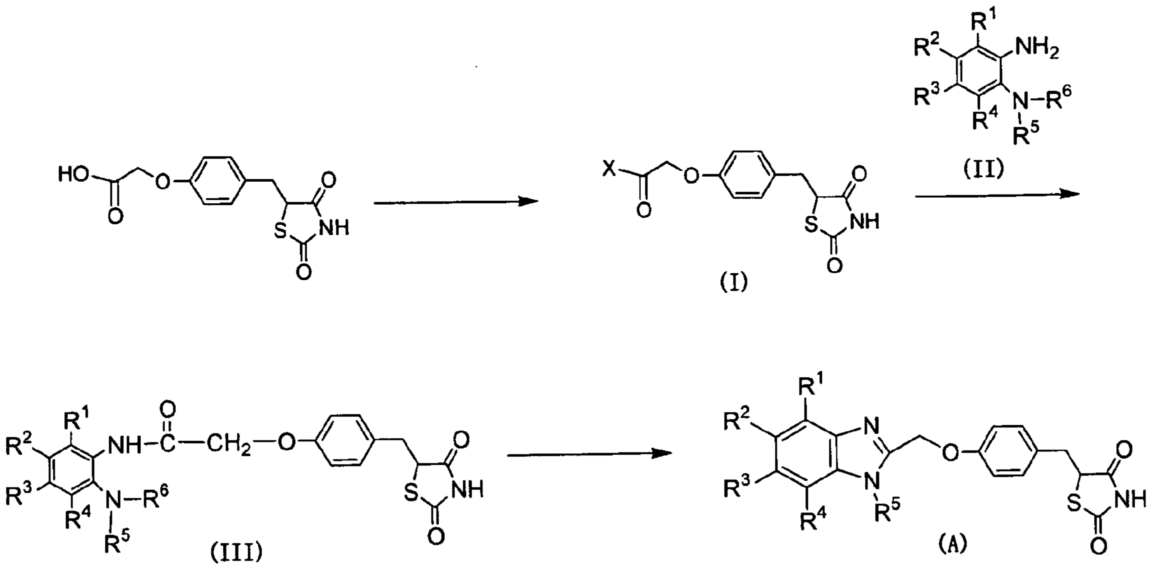

(II)

(I)

(III)

(A)

## Description

### Technical Field

The present invention relates to a high-purity thiazolidinedione compound having excellent prophylactic and therapeutic effects on disease caused by insulin resistance, crystals thereof, a process for producing the same, and a synthetic intermediate therefor.

### Background Art

Thiazolidinedione derivatives are known as compounds having prophylactic and therapeutic effects on diseases caused by insulin resistance such as diabetes mellitus, hyperglycemia, impaired glucose tolerance, hypertension, hyperlipidemia, diabetic complications, gestational diabetes mellitus and polycystic ovary syndrome, and cardiovascular diseases such as atherosclerosis (see Patent Documents 1, 2 and 3, for example).

There are disclosed several processes for producing thiazolidinedione derivatives through different intermediates (see Patent Documents 1 and 4). For example, Patent Document 4 discloses the following production process. (Compound Nos., etc. of formulas in Japanese Patent Laid-Open No. 2001-72671 are herein partially modified, wherein $R^{1B}$ represents a C1-C6 alkyl group, a C1-C6 alkoxy group or the like, $R^{2B}$ represents a C1-C6 alkyl group, and $R^{3B}$ represents a hydrogen atom or a protecting group.)

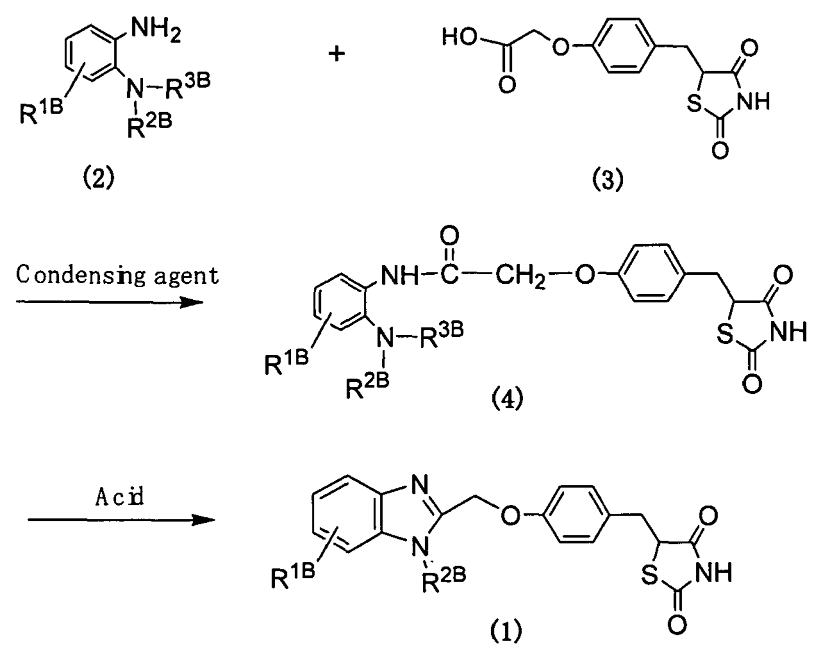


Japanese Patent Laid-0 pen No. 2001-72671

Patent Document 4 describes that the process comprises condensing a 1,2-phenylenediamine derivative represented by the general formula (2) with a compound (3) in the presence of a condensing agent. Patent Document 4 specifically describes a preparation example of a compound represented by the general formula (4), wherein $R^{1B}$ is a methoxy group, $R^{2B}$ is a methyl group, and $R^{3B}$ is a t-butoxycarbonyl group; however, the reaction yield is 85% and there is room for further improvement. Further, complicated operations are needed such as addition of a 5% sodium bicarbonate solution to the reaction mixture and subsequent extraction after completion of the reaction in order to decompose propylphosphonic acid cyclic anhydride excessively used as a condensing agent, and these operations are one reason for the reduced yield and purity.

On the other hand, a production process has never been known heretofore comprising condensing a phenylenediamine compound (the compound (2), for example) with a halide of {4-[(2,4-dioxo-1,3-thiazolidin-5-yl)methyl]phenoxy} acetic acid (the compound (3)). This is because it is reported that when treating a compound having a 1,3-thiazolidine-2,4-dione skeleton like {4-[(2,4-dioxo-1,3-thiazolidin-5-yl)methyl]phenoxy}acetic acid with a halogenating agent such as phosphorus oxychloride, a 2,4-dichlorothiazole derivative is generated (see Non-Patent Documents 1 and 2, for example).

Accordingly, it was considered that when reacting a halogenating agent with (4-[(2,4-dioxo-1,3-thiazolidin-5-yl)methyl] phenoxy}acetic acid, a compound in which the ring part of the thiazole ring is halogenated would similarly be produced as a main product or by-product, not a target acid halide.

Organochlorine solvents such as dichloromethane are generally used as solvents at a laboratory level because of their convenience for use. However, there is a need for a synthesis method not using dichloromethane in an industrial production process using a large amount of a solvent since dichloromethane may be toxic, for example.

[Patent Document 1] Japanese Patent Laid-Open No. 9-95970
[Patent Document 2] Japanese Patent Laid-Open No. 2001-39976
[Patent Document 3] WO 00/71540
[Patent Document 4] Japanese Patent Laid-Open No. 2001-72671
[Non-Patent Document 1] Journal of Chemical Society Perkin Transactions I [J. Chem. Soc. Perkin I] (Britain) 1992, p. 973-978
[Non-Patent Document 2] Bioorganic & Medicinal Chemistry Letters [Bioorganic Med. Chem. Lett.] (Britain) vol. 14, p. 235-238 (2004)

Disclosure of the Invention

An ingredient used in a pharmaceutical is particularly strictly required to have a high purity in order not to cause unexpected side effects (such as toxicity) by impurities in the substance. Further, there is a need to remove impurities in a simpler operation in an industrial production process (mass production process) of the substance.

In addition, it is important to store an active pharmaceutical ingredient for a long time while maintaining its quality. When it is necessary to store the ingredient under low temperature conditions, large-scale refrigeration equipment is necessary to maintain its quality. Therefore, it is industrially significant to find stable crystals that can be stored at room temperature or higher.

As a result of extensive studies to develop a process for producing a thiazolidinedione derivative having excellent prophylactic and therapeutic effects on diseases caused by insulin resistance (preferably diabetes mellitus, hyperglycemia, impaired glucose tolerance, hypertension, hyperlipidemia, diabetic complications, gestational diabetes mellitus and polycystic ovary syndrome, more preferably diabetes mellitus, hyperglycemia and impaired glucose tolerance, and most preferably diabetes mellitus) with high quality in a high yield, in a more industrially advantageous operation, and with lower environmental impacts, the present inventors have found a reaction using {4-[(2,4-dioxo-1,3-thiazolidin-5-yl) methyl]phenoxy}acetic acid halide as a novel synthetic intermediate and a novel process for purifying a synthetic intermediate compound having a higher purity and more excellent stability over time in a high yield without decomposition of the compound, and accordingly have established a process for producing a thiazolidinedione derivative with high quality in a high yield in an industrially advantageous operation. Thus, the present invention has been completed. In addition, the inventors have even established a process in which an organochlorine solvent such as dichloromethane is not used in a series of reactions.

The inventors furthermore have found that crystals of {5-4-[(6-methoxy-1-methyl-1H-benzimidazol-2-yl)methoxy]benzyl}thiazolidine-2,4-dione hydrochloride having a high purity are crystals excellent as an active pharmaceutical ingredient. Thus, the present invention has been completed.

The present invention will be described in detail below.

The present invention is:

(1) A crystal comprising {5-4-[(6-methoxy-1-methyl-1H-benzimidazol-2-yl)methoxy]benzyl}thiazolidine-2,4-dione hydrochloride, **characterized in that** the crystal shows main peaks at interplanar spacings d = 14.29, 7.12, 5.34, 4.97, 4.74, 3.95, 3.85, 3.75, 3.55, 3.51, 3.15, 2.84, 2.76, 2.52 and 2.37 in a powder X-ray diffraction pattern obtained by irradiation with a Cu K$\alpha$ line;
(2) A crystal comprising {5-4-[(6-methoxy-1-methyl-1H-benzimidazol-2-yl)methoxy]benzyl}thiazolidine-2,4-dione hydrochloride having a purity of 98% or more, **characterized in that** the crystal shows main peaks at interplanar spacings d = 14.29, 7.12, 5.34, 4.97, 4.74, 3.95, 3.85, 3.75, 3.55, 3.51, 3.15, 2.84, 2.76, 2.52 and 2.37 in a powder X-ray diffraction pattern obtained by irradiation with a Cu K$\alpha$ line;
(3) A crystal comprising {5-4-[(6-methoxy-1-methyl-1H-benzimidazol-2-yl)methoxy]benzyl}thiazolidine-2,4-dione hydrochloride having a purity of 99% or more, **characterized in that** the crystal shows main peaks at interplanar spacings d = 14.29, 7.12, 5.34, 4.97, 4.74, 3.95, 3.85, 3.75, 3.55, 3.51, 3.15, 2.84, 2.76, 2.52 and 2.37 in a powder X-ray diffraction pattern obtained by irradiation with a Cu K$\alpha$ line;
(4) A thiazolidinedione compound represented by the following general formula (A):

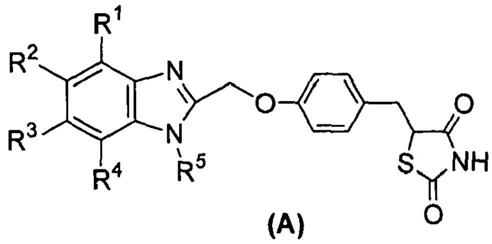

(A)

or a pharmacologically acceptable salt thereof having a purity of 98% or more,
wherein $R^1$, $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, and $R^5$ represents a C1-C6 alkyl group;
(5) {5-4-[(6-Methoxy-1-methyl-1H-benzimidazol-2-yl)methoxy]benzyl}thiazolidine-2,4-dione or a pharmacologically acceptable salt thereof having a purity of 98% or more;
(6) {5-4-[(6-Methoxy-1-methyl-1H-benzimidazol-2-yl)methoxy]benzyl}thiazolidine-2,4-dione or a pharmacologically acceptable salt thereof having a purity of 99% or more;
(7) A compound represented by the following general formula (I):

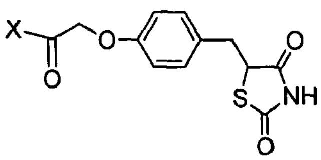

(I)

or a salt thereof,
wherein X represents a halogen atom;
(8) The compound or salt thereof according to claim 7, wherein X is a chlorine atom;
(9) A process for producing a compound represented by the following general formula (I):

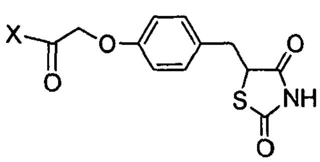

(I)

or a salt thereof,
wherein X represents a halogen atom,
**characterized in that** the process comprises reacting 4-[(2,4-dioxothiazolidin-5-yl)methyl]phenoxyacetic acid with a halogenating agent;
(10) The production process according to (9) above, wherein X is a chlorine atom;
(11) A process for producing a compound represented by the following general formula (III):

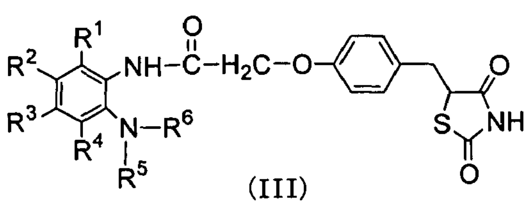

(III)

wherein $R^1$, $R^2$, $R^3$ and $R^4$ are the same or different and each represents a hydrogen atom, a hydroxyl group, a

C1-C6 alkyl group, a C1-C6 alkoxy group, a benzyloxy group, an acetoxy group, a trifluoromethyl group or a halogen atom, $R^5$ represents a C1-C6 alkyl group, and $R^6$ represents a protecting group for an amino group, **characterized in that** the process comprises reacting an acid halide compound represented by the following general formula (I):

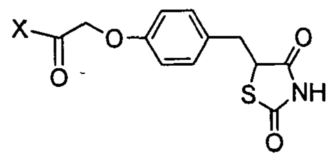

(I)

wherein X represents a halogen atom, with a phenylenediamine compound represented by the following general formula (II):

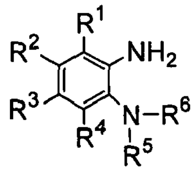

(II)

wherein $R^1$, $R^2$, $R^3$, $R^4$, $R^5$ and $R^6$ are as defined above;

(12) The production process according to (11) above, wherein $R^3$ is a C1-C6 alkoxy group;

(13) A process for producing tert-butyl N-{2-{4-[(2,4-dioxothiazolidin-5-yl)methyl]phenoxyacetylamino}-5-methoxyphenyl}-N-methylcarbamate or a salt thereof, **characterized in that** the process comprises reacting 4-[(2,4-dioxothiazolidin-5-yl)methyl]phenoxyacetyl chloride with tert-butyl N-(2-amino-5-methoxyphenyl)-N-methylcarbamate;

(14) A process for purifying a compound represented by the following general formula (III):

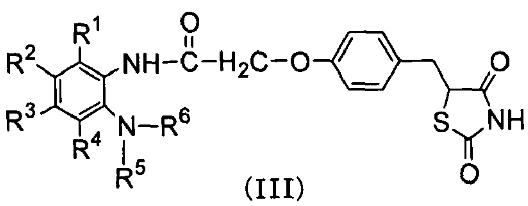

(III)

wherein $R^1$, $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, $R^5$ represents a C1-C6 alkyl group, and $R^6$ represents a protecting group for an amino group, **characterized in that** the process comprises refluxing a suspension or solution of crude crystals of the compound in an organic solvent (wherein the organic solvent is selected from alcohols, ethers, nitriles and mixed solvents thereof);

(15) The purification process according to (14) above, wherein the organic solvent is an alcoholic solvent;

(16) The purification process according to (14) or (15) above, wherein the compound represented by the general formula (III) is tert-butyl N-{2-{4-[(2,4-dioxothiazolidin-5-yl)methyl]phenoxyacetylamino}-5-methoxyphenyl}-N-methylcarbamate or a salt thereof;

(17) A process for producing a thiazolidinedione compound represented by the following general formula (A):

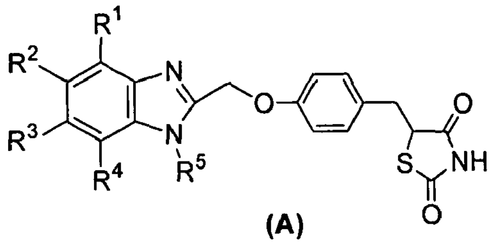

(A)

or a pharmacologically acceptable salt thereof,
wherein $R^1$, $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, and $R^5$ represents a C1-C6 alkyl group,
**characterized in that** the process comprises refluxing a suspension or solution of a compound represented by the following general formula (III):

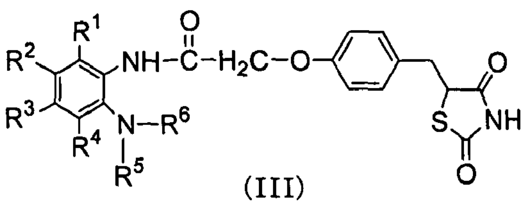

(III)

wherein $R^1$, $R^2$, $R^3$, $R^4$ and $R^5$ are as defined above, and $R^6$ represents a protecting group for an amino group, in an organic solvent (wherein the organic solvent is selected from alcohols, ethers, nitriles and mixed solvents thereof) so that the final product has an impurity content of 2% or less;
(18) A process for producing a thiazolidinedione compound represented by the following general formula (A):

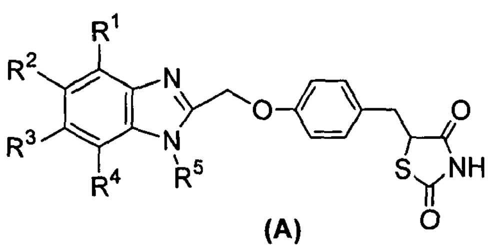

(A)

or a pharmacologically acceptable salt thereof,
wherein $R^1$, $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, and $R^5$ represents a C1-C6 alkyl group,
**characterized in that** the process comprises reacting a compound represented by the following general formula (I) :

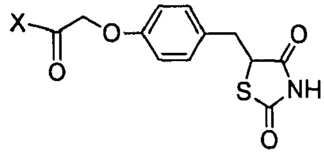

(I)

wherein X represents a halogen atom, with a compound represented by the following general formula (II):

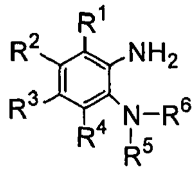

(II)

wherein $R^1$, $R^2$, $R^3$, $R^4$ and $R^5$ are as defined above, and $R^6$ represents a protecting group for an amino group; and treating with an acid the resulting compound represented by the following general formula (III):

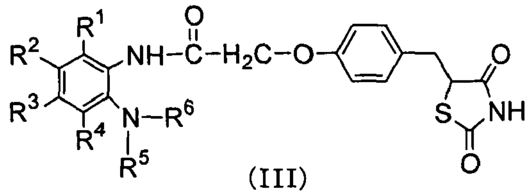

(III)

wherein $R^1$, $R^2$ , $R^3$ , $R^4$ , $R^5$ and $R^6$ are as defined above;
(19) A process for producing a thiazolidinedione compound represented by the following general formula (A):

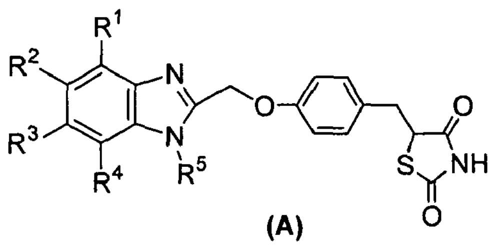

**(A)**

or a pharmacologically acceptable salt thereof,
wherein $R^1$, $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, and $R^5$ represents a C1-C6 alkyl group,
**characterized in that** the process comprises reacting 4-[(2,4-dioxothiazolidin-5-yl)methyl]phenoxyacetic acid with a halogenating agent; reacting the resulting compound represented by the following general formula (I) :

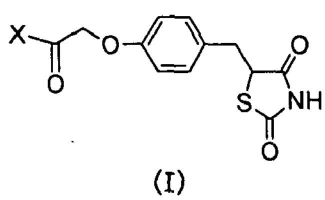

(I)

or salt thereof,
wherein X represents a halogen atom, with a compound represented by the following general formula (II):

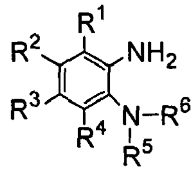

(II)

wherein $R^1$, $R^2$, $R^3$, $R^4$ and $R^5$ are as defined above, and $R^6$ represents a protecting group for an amino group, to obtain crude crystals of a compound represented by the following general formula (III):

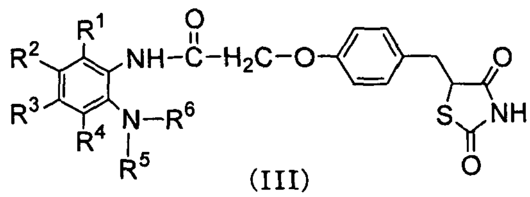

(III)

wherein $R^1$ $R^2$ , $R^3$ , $R^4$ , $R^5$ and $R^6$ are as defined above; refluxing a suspension or solution of the crude crystals in an organic solvent (wherein the organic solvent is selected from alcohols, ethers, nitriles and mixed solvents thereof) to purify the compound; and subsequently cyclizing the compound into an imidazole ring by treatment with an acid;
(20) The production process according to (17) to (19) above, wherein the thiazolidinedione compound represented by the general formula (A) is {5-4-[(6-methoxy-1-methyl-1H-benzimidazol-2-yl)methoxy]benzyl}thiazolidine-2,4-dione or a pharmacologically acceptable salt thereof;
(21) A thiazolidinedione compound represented by the general formula (A):

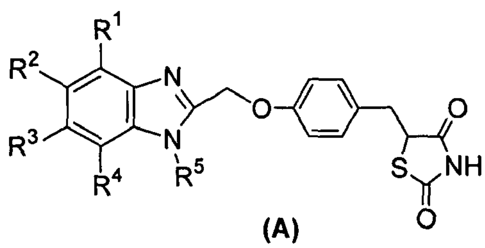

**(A)**

or a pharmacologically acceptable salt thereof,
wherein $R^1$ $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, and $R^5$ represents a C1-C6 alkyl group,
which is obtained by reacting 4-[(2,4-dioxothiazolidin-5-yl)methyl]phenoxyacetic acid with a halogenating agent; reacting the resulting compound represented by the following general formula (I):

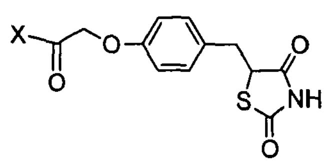

(I)

or salt thereof,
wherein X represents a halogen atom, with a compound represented by the following general formula (II):

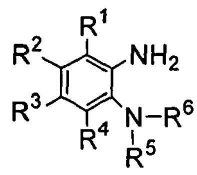

(II)

wherein $R^1$, $R^2$, $R^3$, $R^4$ and $R^5$ are as defined above, and $R^6$ represents a protecting group for an amino group, to obtain a compound represented by the following general formula (III):

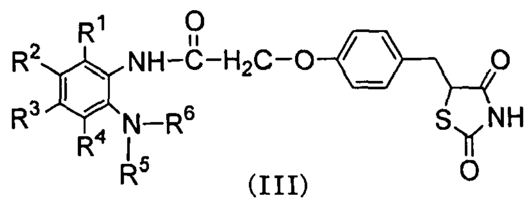

(III)

or a salt thereof,
wherein $R^1$, $R^2$, $R^3$, $R^4$, $R^5$ and $R^6$ are as defined above; refluxing a suspension or solution of the product in an organic solvent (wherein the organic solvent is selected from alcohols, ethers, nitriles and mixed solvents thereof) to purify the product; and subsequently cyclizing the product into an imidazole ring by treatment with an acid;
(22) The thiazolidinedione compound or pharmacologically acceptable salt thereof according to (21) above, wherein the thiazolidinedione compound represented by the general formula (A) is {5-4-[(6-methoxy-1-methyl-1H-benzimidazol-2-yl)methoxy]benzyl}thiazolidine-2,4-dione;
(23) A pharmaceutical composition comprising the crystal according to (1) to (3) above as an active ingredient;
(24) A pharmaceutical composition comprising the crystal according to (1) to (3) above as an active ingredient for prevention or treatment of diabetes mellitus, hyperglycemia, impaired glucose tolerance, hypertension, hyperlipidemia, diabetic complications, gestational diabetes mellitus, polycystic ovary syndrome or atherosclerosis;
(25) A pharmaceutical composition comprising the crystal according to (1) to (3) above as an active ingredient for prevention or treatment of diabetes mellitus;
(26) A pharmaceutical composition comprising the compound or pharmacologically acceptable salt thereof according to (4) to (6) above;
(27) A pharmaceutical composition comprising the compound or pharmacologically acceptable salt thereof according to (4) to (6) above for prevention or treatment of diabetes mellitus, hyperglycemia, impaired glucose tolerance, hypertension, hyperlipidemia, diabetic complications, gestational diabetes mellitus, polycystic ovary syndrome or atherosclerosis;
(28) A pharmaceutical composition comprising the compound or pharmacologically acceptable salt thereof according to (4) to (6) above for prevention or treatment of diabetes mellitus;
(29) A pharmaceutical composition comprising the compound or pharmacologically acceptable salt thereof according to (21) or (22) above;
(30) A pharmaceutical composition comprising the compound or pharmacologically acceptable salt thereof according to (21) or (22) above for prevention or treatment of diabetes mellitus, hyperglycemia, impaired glucose tolerance, hypertension, hyperlipidemia, diabetic complications, gestational diabetes mellitus, polycystic ovary syndrome or atherosclerosis;
(31) A pharmaceutical composition comprising the compound or pharmacologically acceptable salt thereof according to (21) or (22) above for prevention or treatment of diabetes mellitus;
(32) Use of the crystal according to (1) to (3) above for production of a pharmaceutical composition for prevention or treatment of diabetes mellitus;
(33) Use of the compound or pharmacologically acceptable salt thereof according to (4) to (6) above for production of a pharmaceutical composition for prevention or treatment of diabetes mellitus;
(34) Use of the compound or pharmacologically acceptable salt thereof according to (21) or (22) above for production of a pharmaceutical composition for prevention or treatment of diabetes mellitus;
(35) A method for preventing or treating diabetes mellitus, **characterized in that** the method comprises administering the crystal according to (1) to (3) above;

(36) A method for preventing or treating diabetes mellitus, **characterized in that** the method comprises administering the compound or pharmacologically acceptable salt thereof according to (4) to (6) above;

(37) A method for preventing or treating diabetes mellitus, **characterized in that** the method comprises administering the compound or pharmacologically acceptable salt thereof according to (21) or (22) above;

(38) A composition of crystals of {5-4-[(6-methoxy-1-methyl-1H-benzimidazol-2-yl)methoxy]benzyl}thiazolidine-2,4-dione hydrochloride having a content of {5-4-[(6-methoxy-1-methyl-1H-benzimidazol-2-yl)methoxy]benzyl}thiazolidine-2,4-dione hydrochloride of 98% or more excluding water, **characterized in that** the composition shows main peaks at interplanar spacings d = 14.29, 7.12, 5.34, 4.97, 4.74, 3.95, 3.85, 3.75, 3.55, 3.51, 3.15, 2.84, 2.76, 2.52 and 2.37 in a powder X-ray diffraction pattern obtained by irradiation with a Cu K$\alpha$ line;

(39) A composition of crystals of {5-4-[(6-methoxy-1-methyl-1H-benzimidazol-2-yl)methoxy]benzyl}thiazolidine-2,4-dione hydrochloride having a content of {5-4-[(6-methoxy-1-methyl-1H-benzimidazol-2-yl)methoxy]benzyl}thiazolidine-2,4-dione hydrochloride of 99% or more excluding water, **characterized in that** the composition shows main peaks at interplanar spacings d = 14.29, 7.12, 5.34, 4.97, 4.74, 3.95, 3.85, 3.75, 3.55, 3.51, 3.15, 2.84, 2.76, 2.52 and 2.37 in a powder X-ray diffraction pattern obtained by irradiation with a Cu K$\alpha$ line;

(40) A composition of purified {5-4-[(6-methoxy-1-methyl-1H-benzimidazol-2-yl)methoxy]benzyl}thiazolidine-2,4-dione hydrochloride, which has a content of {5-4-[(6-methoxy-1-methyl-1H-benzimidazol-2-yl)methoxy]benzyl}thiazolidine-2,4-dione hydrochloride of 98 wt% or more excluding water; and

(41) A composition of purified {5-4-[(6-methoxy-1-methyl-1H-benzimidazol-2-yl)methoxy]benzyl}thiazolidine-2,4-dione hydrochloride, which has a content of {5-4-[(6-methoxy-1-methyl-1H-benzimidazol-2-yl)methoxy]benzyl}thiazolidine-2,4-dione hydrochloride of 99 wt% or more excluding water.

The "purity" in the present invention is a value measured by quantitative analysis.

The "C1-C6 alkyl group" in the present invention is a linear or branched alkyl group having 1 to 6 carbon atoms. Examples of such a group include a methyl group, ethyl group, propyl group, isopropyl group, butyl group, isobutyl group, s-butyl group, t-butyl group, pentyl group, isopentyl group, 2-methylbutyl group, neopentyl group, 1-ethylpropyl group, hexyl group, 4-methylpentyl group, 3-methylpentyl group, 2-methylpentyl group, 1-methylpentyl group, 3,3-dimethylbutyl group, 2,2-dimethylbutyl group, 1,1-dimethylbutyl group, 1,2-dimethylbutyl group, 1,3-dimethylbutyl group, 2,3-dimethylbutyl group and 2-ethylbutyl group. For $R^1$, $R^2$, $R^3$, $R^4$ and $R^5$, a C1-C4 alkyl group is preferable, an ethyl or methyl group is more preferable, and a methyl group is particularly preferable.

The "C1-C6 alkoxy group" in the present invention is a group in which the C1-C6 alkyl group is bonded to an oxygen atom. Examples of such a group include a methoxy group, ethoxy group, propoxy group, isopropoxy group, butoxy group, isobutoxy group, s-butoxy group, t-butoxy group, pentoxy group, isopentoxy group, 2-methylbutoxy group, neopentoxy group, 1-ethylpropoxy group, hexyloxy group, 4-methylpentoxy group, 3-methylpentoxy group, 2-methylpentoxy group, 3,3-dimethylbutoxy group, 2,2-dimethylbutoxy group, 1,1-dimethylbutoxy group, 1,2-dimethylbutoxy group, 1,3-dimethylbutoxy group, 2,3-dimethylbutoxy group and 2-ethylbutoxy group. For $R^1$, $R^2$, $R^3$ and $R^4$, a C1-C4 alkoxy group is preferable, an ethoxy or methoxy group is more preferable, and a methoxy group is particularly preferable.

The "halogen atom" in the present invention is a fluorine, chlorine, bromine or iodine atom. For $R^1$, $R^2$, $R^3$, $R^4$ and X, a fluorine, chlorine or bromine atom is preferable, and chlorine is more preferable.

The "protecting group for an amino group" in the present invention is not specifically limited so long as it is usually used as a protecting group for an amino group. Examples of such a group include arylmethyl groups which may be substituted with C1-C6 alkyl, C1-C6 alkoxy or halogen such as a trityl group, monomethoxytrityl group, dimethoxytrityl group, trimethoxytrityl group, benzyl group, methylbenzyl group, methoxybenzyl group, chlorobenzyl group, bromobenzyl group and naphthylmethyl group; arylmethyloxycarbonyl groups which may be substituted with C1-C6 alkyl, C1-C6 alkoxy or halogen such as a benzyloxycarbonyl group, methylbenzyloxycarbonyl group, methoxybenzyloxycarbonyl group, chlorobenzyloxycarbonyl group, bromobenzyloxycarbonyl group and naphthylmethyloxycarbonyl group; alkenyloxycarbonyl groups such as an allyloxycarbonyl group and methallyloxycarbonyl group; and alkyloxycarbonyl groups such as a t-butoxycarbonyl group. For $R^6$, a t-butoxycarbonyl group, benzyl group, p-methoxybenzyl group, p-bromobenzyl group, benzyloxycarbonyl group, p-methoxybenzyloxycarbonyl group, p-bromobenzyloxycarbonyl group or allyloxycarbonyl group is preferable, a t-butoxycarbonyl group, benzyloxycarbonyl group, p-methoxybenzyloxycarbonyl group, p-bromobenzyloxycarbonyl group or allyloxycarbonyl group is more preferable, and a t-butoxycarbonyl group is particularly preferable.

$R^1$ is preferably a hydrogen atom or a C1-C4 alkoxy group, more preferably a hydrogen atom or a methoxy group, and particularly preferably a hydrogen atom.

$R^2$ is preferably a hydrogen atom or a C1-C4 alkoxy group, more preferably a hydrogen atom or a methoxy group, and particularly preferably a hydrogen atom.

$R^3$ is preferably a hydrogen atom or a C1-C4 alkoxy group, more preferably a hydrogen atom or a methoxy group, and particularly preferably a methoxy group.

$R^4$ is preferably a hydrogen atom or a C1-C4 alkoxy group, more preferably a hydrogen atom or a methoxy

group, and particularly preferably a hydrogen atom.

$R^5$ is preferably a C1-C4 alkyl group, more preferably a C1-C2 alkyl group, and particularly preferably a methyl group.

$R^6$ is preferably a t-butoxycarbonyl group, benzyloxycarbonyl group, p-methoxybenzyloxycarbonyl group or p-bromobenzyloxycarbonyl group, and more preferably a t-butoxycarbonyl group.

X is preferably a chlorine atom.

The process for producing a synthetic intermediate (I), a synthetic intermediate (III) and a final target compound (A) and a salt thereof according to the present invention will be described in detail below.

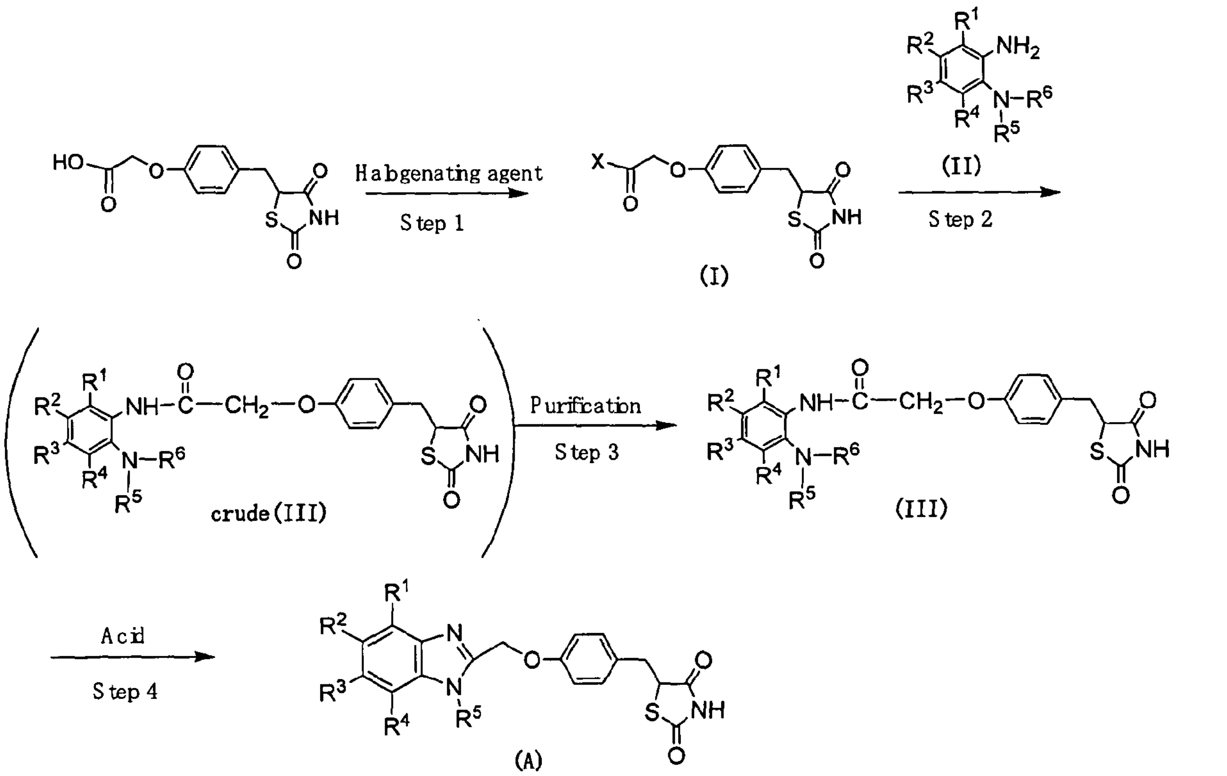


The process of the present invention comprises Step 1 of reacting 4-[(2,4-dioxothiazolidin-5-yl)methyl]phenoxyacetic acid with a halogenating agent to produce an acid halide represented as a compound (I); Step 2 of reacting the compound (I) with a compound (II) to produce a compound (III); Step 3 of purifying the compound (III); and Step 4 of converting the compound (III) into a compound (A) in the presence of an acid. Each step will be described in detail below.

(Step 1)

This step is a step of producing an acid halide (I) and is effected by reacting 4-[(2,4-dioxothiazolidin-5-yl)methyl]phenoxyacetic acid with a halogenating agent in an inert solvent.

The halogenating agent used in this step is not specifically limited so long as it can convert a carboxylic acid into an acid halide. Examples of such a halogenating agent include thionyl chloride, thionyl bromide, oxalyl chloride, phosphorus oxychloride, phosphorus trichloride and phosphorus pentachloride. The halogenating agent is preferably thionyl chloride, oxalyl chloride, phosphorus pentachloride or the like, and is particularly preferably thionyl chloride.

The amount of the halogenating agent used in this step is not specifically limited so long as the amount is 1 equivalent or more based on 4-[(2,4-dioxothiazolidin-5-yl)methyl]phenoxyacetic acid used, but such an amount is preferably 1 to 2 equivalents, and more preferably 1 to 1.2 equivalents.

In this step, the reaction is usually carried out in a solvent. When used, the solvent is not specifically limited so long as it does not inhibit the reaction. Examples of such a solvent include aliphatic hydrocarbons such as hexane, heptane, ligroin and petroleum ether; aromatic hydrocarbons such as benzene, toluene and xylene; nitriles such as acetonitrile, propionitrile and benzonitrile; halogenated hydrocarbons such as dichloromethane, chloroform, 1,2-dichloroethane and carbon tetrachloride; ethers such as diethyl ether, diisopropyl ether, tetrahydrofuran, dioxane, dimethoxyethane and diethylene glycol dimethyl ether; amides such as formamide, dimethylformamide, dimethylacetamide and

hexamethylphosphoric triamide; sulfoxides such as dimethyl sulfoxide; sulfolane; and mixtures thereof. The solvent is preferably a halogenated hydrocarbon, a nitrile, an ether, an amide or a mixture thereof, more preferably acetonitrile, dichloromethane, chloroform, tetrahydrofuran, dimethylformamide or a mixture thereof, and particularly preferably dichloromethane or acetonitrile.

In this step, the reaction may proceed more rapidly by addition of a catalyst.

When the catalyst is added, an amine, amine derivative or nitrogen-containing heterocyclic compound is usually used as the catalyst. The amine used is usually a tertiary amine. Examples of such an amine include trialkylamines such as trimethylamine, triethylamine, diisopropylethylamine and tributylamine; dialkylarylamines such as N,N-dimethylaniline and N,N-diethylaniline; and diarylalkylamines such as diphenylmethylamine. Examples of the amine derivative include N,N-dialkylamides such as dimethylformamide and dimethylacetamide. Examples of the nitrogen-containing heterocyclic compound include pyridine, N,N-dimethyl-4-aminopyridine, imidazole and triazole. The catalyst is preferably trimethylamine, triethylamine, diisopropylethylamine, tributylamine, N,N-dimethylaniline, dimethylformamide, dimethylacetamide, pyridine or N,N-dimethyl-4-aminopyridine, more preferably triethylamine, dimethylformamide, pyridine or N,N-dimethyl-4-aminopyridine, and particularly preferably dimethylformamide.

The amount of the catalyst used is not specifically limited, but is usually 0.1 to 20 equivalents, preferably 0.1 to 10 equivalents, and more preferably 0.3 to 5 equivalents based on the halogenating agent used. The reaction temperature in this step varies depending on the raw material compound, reagent and the like, but is usually -20˚C to 150˚C, preferably -10˚C to 100˚C, and more preferably -10 to 40˚C.

The reaction time in this step varies depending on the raw material compound, reagent, reaction temperature and the like, but is usually 30 minutes to 80 hours, preferably 30 minutes to 48 hours, and more preferably 1 hour to 6 hours.

After completion of this step, Step 2 may be carried out after or without isolation of the compound (I) or a salt thereof; Step 2 is preferably performed without isolation.

(Step 2)

This step is a step of producing a compound (III) and is effected by reacting the acid halide (I) with a known phenylenediamine compound (II) in an inert solvent.

In this step, the reaction may proceed rapidly by use of a base. Examples of the base used in this step include alkali metal carbonates such as lithium carbonate, sodium carbonate and potassium carbonate; alkali metal bicarbonates such as lithium bicarbonate, sodium bicarbonate and potassium bicarbonate; alkali metal hydrides such as lithium hydride, sodium hydride and potassium hydride; alkali metal hydroxides such as lithium hydroxide, sodium hydroxide and potassium hydroxide; alkali metal alkoxides such as lithium methoxide, sodium methoxide, sodium ethoxide and potassium t-butoxide; and organic amines such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO) and 1,8-diazabicyclo[5.4.0]-7-undecene (DBU). The base is preferably an organic amine, more preferably triethylamine, tributylamine or pyridine, and particularly preferably triethylamine.

This step is usually carried out in a solvent. The solvent is not specifically limited so long as it does not inhibit the reaction. Examples of such a solvent include aliphatic hydrocarbons such as hexane, heptane, ligroin and petroleum ether; aromatic hydrocarbons such as benzene, toluene and xylene; nitriles such as acetonitrile, propionitrile and benzonitrile; halogenated hydrocarbons such as dichloromethane, chloroform, 1,2-dichloroethane and carbon tetrachloride; ethers such as diethyl ether, diisopropyl ether, tetrahydrofuran, dioxane, dimethoxyethane and diethylene glycol dimethyl ether; amides such as formamide, dimethylformamide, dimethylacetamide and hexamethylphosphoric triamide; sulfoxides such as dimethyl sulfoxide; sulfones such as sulfolane; and mixtures thereof. The solvent is preferably a halogenated hydrocarbon, a nitrile, an ether, an amide or a mixture thereof, more preferably acetonitrile, dichloromethane, chloroform, tetrahydrofuran, dimethylformamide or a mixture thereof, and particularly preferably dichloromethane or acetonitrile.

The reaction temperature in this step varies depending on the raw material compound, reagent and the like, but is usually -20˚C to 150˚C, and preferably -20˚C to 100˚C.

The reaction time in this step varies depending on the raw material compound, reagent, reaction temperature and the like, but is usually 30 minutes to 80 hours. The reaction time is preferably 1 hour to 48 hours.

After completion of the reaction in this step, the compound (III) is isolated by isolation operations such as natural crystallization or extraction and concentration following common post-treatment, and thereafter Step 3 or 4 is carried out.

(Step 3)

This step is a step of producing a compound (III) having a higher purity by heating and stirring the compound (III) in an organic solvent to dissolve or suspend the compound in the solvent, and then cooling the solution or suspension to remove impurities by recrystallization or resuspension; however, this step may be omitted when the compound (III) is not crystalline or has a sufficiently high purity.

This step is carried out in a solvent. The solvent is not specifically limited so long as it does not react with the compound (III) and dissolves the compound to a certain extent. Examples of such a solvent include alcohols such as methanol, ethanol, propanol, isopropyl alcohol and butyl alcohol; nitriles such as acetonitrile, propionitrile and benzonitrile; ethers such as diethyl ether, diisopropyl ether, tetrahydrofuran, dioxane, dimethoxyethane and diethylene glycol dimethyl ether; and mixtures thereof. The solvent is preferably acetonitrile, methanol, ethanol or a mixture thereof, and particularly preferably methanol.

The heating temperature in this step varies depending on the properties of the compound (III) and the solvent, but is usually room temperature to a reflux temperature of the solvent used, and is preferably a reflux temperature of the solvent used.

The time in this step varies depending on the properties of the compound (III) and the solvent, but is usually 10 minutes to 20 hours, and is preferably 20 minutes to 10 hours.

After completion of this step, the compound (III) is isolated by a filtration operation following a common crystallization operation, and thereafter Step 4 is carried out.

(Step 4)

This step is a step of producing a final target compound (A) and is effected by cyclizing the compound (III) in the presence of an acid.

This step is carried out in a solvent. The solvent is not specifically limited so long as it does not react with the compound (III) and dissolves the compound to a certain extent. Examples of such a solvent include aliphatic hydrocarbons such as hexane, heptane, ligroin and petroleum ether; aromatic hydrocarbons such as benzene, toluene and xylene; alcohols such as methanol, ethanol, propanol, isopropyl alcohol and butyl alcohol; nitriles such as acetonitrile, propionitrile and benzonitrile; halogenated hydrocarbons such as dichloromethane, chloroform, 1,2-dichloroethane and carbon tetrachloride; ethers such as diethyl ether, diisopropyl ether, tetrahydrofuran, dioxane, dimethoxyethane and diethylene glycol dimethyl ether; and mixtures thereof. The solvent is preferably a halogenated hydrocarbon, a nitrile, an alcohol, an ether or a mixture thereof, more preferably dichloromethane, chloroform, acetonitrile, methanol, ethanol or a mixture thereof, and particularly preferably methanol.

The acid used in this step may be either a Bronsted acid or a Lewis acid, but is usually a Bronsted acid. The Bronsted acid usually used in this step is not specifically limited. Examples of such an acid include inorganic acids such as hydrogen chloride, hydrogen bromide, hydrochloric acid, bromic acid, sulfuric acid, nitric acid, phosphoric acid and boric acid; and organic acids such as formic acid, acetic acid, propionic acid, oxalic acid, succinic acid, maleic acid, hydroxyacetic acid, fumaric acid, citric acid, tartaric acid, benzoic acid and salicylic acid. The Bronsted acid is preferably hydrochloric acid, sulfuric acid, acetic acid, citric acid or tartaric acid, more preferably hydrochloric acid or sulfuric acid, and particularly preferably hydrochloric acid.

When the compound (A) is produced as a salt in this step, a corresponding salt may be produced as is, using an acid of the salt to be produced.

The amount of the acid used in this step is not specifically limited so long as the amount is 1 equivalent or more based on the compound (III) used, but the amount is preferably 1 to 15 equivalents, and more preferably 2 to 8 equivalents.

When the compound (III) is dissolved in the solvent in this step, inorganic or organic insoluble impurities included in the compound (III) may be removed by performing a filtration operation prior to addition of the acid.

The reaction temperature in this step varies depending on the properties of the compound (III), the acid and the solvent, but is usually room temperature to 100°C, preferably 35°C to 80°C, and more preferably 40°C to 60°C.

The reaction time in this step varies depending on the properties of the compound (III), the acid and the solvent, but is usually 30 minutes to 20 hours, and is preferably 1 hour to 10 hours.

After completion of the reaction in this step, the compound (A) or salt thereof may be isolated by a filtration operation when crystallized as crystals. The compound (A) or salt thereof may be isolated by an isolation operation such as an extraction operation following common post-treatment when not crystallized. The resulting compound (A) may be used as is after drying, or may be used after carrying out a common purification operation such as recrystallization or column chromatography.

A medicine containing the high-purity thiazolidinedione compound of the present invention and crystals thereof can be used in a formulation that can be orally administered such as tablets, capsules, pills, granules or fine granules, and preferably tablets.

The formulation may optionally contain a pharmaceutically acceptable additive. Examples of such an additive may include excipients (for example, sugar derivatives such as lactose, saccharose, glucose, mannitol and sorbitol; starch derivatives such as corn starch, potato starch, pregelatinized starch, dextrin, carboxymethyl starch and sodium carboxymethyl starch; pregelatinized starch; cellulose derivatives such as crystalline cellulose, methylcellulose, hydroxypropylcellulose, low-substituted hydroxypropylcellulose, hydroxypropylmethylcellulose, carmellose, carmellose calcium, croscarmellose and croscarmellose sodium; gum arabic; dextran; pullulan; silicate derivatives such as light anhydrous silicic acid, calcium silicate, silicic acid hydrate, synthetic aluminum silicate and magnesium aluminometasilicate; phosphate derivatives such as dicalcium phosphate; chloride derivatives such as sodium chloride; carbonate derivatives such as calcium carbonate; sulfate derivatives such as calcium sulfate; and mixtures thereof), binders (for example, compounds exemplified as the excipients; gelatin; polyvinylpyrrolidone; macrogol; and mixtures thereof), disintegrants (for example, compounds exemplified as the excipients; crosslinked polyvinylpyrrolidone; and mixtures thereof), lubricants (for example, stearic acid; metal salts of stearic acid such as calcium stearate and magnesium stearate; metal salts of benzoic acid such as sodium benzoate; waxes such as veegum and spermaceti; boric acid; glycols; carboxylic acids such as fumaric acid and adipic acid; metal salts of sulfuric acid such as sodium sulfate; leucine; metal salts of laurylsulfuric acid such as sodium lauryl sulfate and magnesium lauryl sulfate; silicate derivatives exemplified as the excipients; starch derivatives exemplified as the excipients; hydrogenated vegetable oils; carnauba wax; sucrose fatty acid ester; and mixtures thereof), stabilizers (for example, benzoic acid; metal salts of benzoic acid such as sodium benzoate; p-hydroxybenzoates such as methylparaben and propylparaben; alcohols such as chlorobutanol, benzyl alcohol and phenylethyl alcohol; benzalkonium chloride; phenols such as phenol and cresol; thimerosal; acetic anhydride; sorbic acid; and mixtures thereof), fluidizers (for example, silicate derivatives exemplified as the excipients; talc; and mixtures thereof), surfactants (for example, polysolvates such as polysolvate 80; polyoxyethylene hydrogenated castor oils such as polyoxyethylene hydrogenated castor oil 60; sorbitan fatty acid esters; sucrose fatty acid esters; polyoxyethylene polyoxypropylene glycols; polyoxyethylene fatty acid ethers; polyoxyl stearates; and mixtures thereof, preferably polysolvate 80, polyoxyethylene hydrogenated castor oil 60 and a mixture thereof), colorants (for example, yellow iron sesquioxide, iron sesquioxide and black iron oxide), antioxidants, correctives (for example, commonly used sweeteners, acidulants and flavors) and diluents. The type and amount of the additive used vary depending on the tablets, capsules or other drug administration modes, but are selected based on a known technique in the field of formulation.

For example, in the case of tablets, the content of the binder is usually 1 to 10 parts by weight (preferably 2 to 5 parts by weight), the content of the disintegrant is usually 1 to 40 parts by weight (preferably 5 to 30 parts by weight), the content of the lubricant is usually 0.1 to 10 parts by weight (preferably 0.5 to 3 parts by weight), and the content of the fluidizer is usually 0.1 to 10 parts by weight (preferably 0.5 to 5 parts by weight) based on the total pharmaceutical composition.

The pharmaceutical composition of the present invention is easily produced by a known method (for example, a kneading method using water or a wet granulation method) using a pharmaceutically acceptable additive. In an example of such production, an active ingredient, a stabilizer, an excipient, a binder, a disintegrant and, as necessary, another adjuvant or the like are mixed in a high-speed stirring granulator, and the resulting mixture is kneaded with a binder solution to obtain a granulated product. The resulting granulated product is dried in a fluid bed dryer, the dried granulated product is forced to pass through a screen using a crushing granulator and mixed with a lubricant, a disintegrant and, as necessary, another adjuvant or the like in a V-type mixer, and the resulting mixture is tableted or capsulated, so that tablets or capsules can be produced, respectively.

The resulting tablets can be sugar-coated or coated (preferably coated) as necessary. For example, the resulting tablets can be film-coated by spraying a coating solution made of hydroxypropylmethylcellulose, talc, titanium oxide, lactose, triacetin or polyethylene glycol; yellow iron sesquioxide or iron sesquioxide; and water over the tablets in a pan coater.

Alternatively, the above kneaded product obtained by mixing in a high-speed stirring granulator and kneading with a binder solution is converted into a granulated product using an extrusion granulator and then dried in a fence-type dryer, and the dried granulated product is forced to pass through a screen using a crushing granulator, so that granules can be produced.

The pharmaceutical composition of the present invention can be administered to warm-blooded animals (particularly humans). The dose of 5-[4-(6-methoxy-1-methyl-1H-benzimidazol-2-ylmethoxy)benzyl]thiazolidine-2,4-dione or a pharmacologically acceptable salt thereof as an active ingredient may vary depending on various conditions such as the symptoms, age and body weight of the patient, but the active ingredient can be orally administered to the human in one to six doses per day at 0.1 mg/body to 20 mg/body (preferably 0.5 mg/body to 3 mg/body) per dose according to the symptoms, for example.

The present invention provides crystals of a thiazolidinedione derivative having excellent prophylactic and therapeutic effects on a known disease caused by insulin resistance and a process for producing the thiazolidinedione derivative suitable for mass synthesis. When the production process of the present invention is used, the target compound can be produced with a high purity in a high yield, in a simple operation using an inexpensive reagent, and with reduced environmental burdens even in the case of mass synthesis.

Brief Description of the Drawing

Figure 1 is a powder X-ray diffraction pattern of crystals of {5-4-[(6-methoxy-1-methyl-1H-benzimidazol-2-yl)methoxy]benzyl}thiazolidine-2,4-dione hydrochloride obtained by irradiation with a Cu K$\alpha$ line (wavelength $\lambda$ = 1.54 Å).

The vertical axis for the powder X-ray diffraction pattern indicates a diffraction intensity in a count per second (cps) unit, and the horizontal axis indicates a 2θ value.

Best Mode for Carrying Out the Invention

The present invention will be described in more detail below with reference to the examples and the like; however, the scope of the present invention is not limited thereto.

Examples

(Example 1)

4-[(2,4-Dioxothiazolidin-5-yl)methyl]phenoxyacetyl chloride

Thionyl chloride (170 mg, 1.34 mmol) and then pyridine (1 drop) were added to a suspension of 4-[(2,4-dioxothiazolidin-5-yl)methyl]phenoxyacetic acid (220 mg, 0.78 mmol) in dichloromethane (10 ml) at room temperature, and the mixture was refluxed for 3.5 hours. The resulting solution was concentrated under reduced pressure to obtain about 250 mg of the gummy target compound.

Nuclear magnetic resonance spectrum (400 MHz, DMSO-$d_6$) δ (ppm): 3.05 (1H, dd, J = 9.0 Hz, J = 14.1 Hz, $CH_2CH$), 3.31 (1H, dd, J = 4.1 Hz, J = 14.1 Hz, $CH_2CH$), 4.64 (2H, s, $CH_2O$), 4.87 (1H, dd, J = 4.1 Hz, J = 9.0 Hz, $CH_2CH$), 6.85 (2H, d, J = 8.6 Hz, aromatic), 7.16 (2H, J = 8.6 Hz, aromatic), 12.02 (1H, s, NH).

(Example 2)

tert-Butyl N-{2-{4-[(2,4-dioxothiazolidin-5-yl)methyl]phenoxyacetylamino}-5-methoxyphenyl}-N-methylcarbamate

Thionyl chloride (27.66 g, 232.5 mmol) and dimethylformamide (12 ml) were poured into a suspension of 4-[(2,4-dioxothiazolidin-5-yl)methyl]phenoxyacetic acid (60.0 g, 213.3 mmol) in dichloromethane (390 ml), and the mixture was heated to reflux (39˚C). After completion of dissolution, the solution was stirred for 30 minutes and cooled to 0 to 5˚C. A solution of tert-butyl N-(2-amino-5-methoxyphenyl)-N-methylcarbamate (53.84 g, 213.4 mmol) and triethylamine (25.92 g, 256.2 mmol) in dichloromethane (624 ml) was added dropwise while maintaining the internal temperature at 5˚C or less. The reaction solution was stirred at 5˚C for one hour. Then, dichloromethane (300 ml) was poured in, followed by addition of a solution prepared from sodium bicarbonate (24 g) and water (480 ml). The mixture was stirred at 20˚C for 20 minutes, allowed to stand, and then separated, and the aqueous layer was discarded. Water (480 ml) was added to the organic layer, the mixture was stirred at 20˚C for 20 minutes, allowed to stand, and then separated, and the aqueous layer was discarded. A solution of 38% hydrochloric acid (19.8 ml) and water (480 ml) was poured into the organic layer. The mixture was stirred at 20˚C for 20 minutes, and then the aqueous layer was discarded. Activated carbon (1.8 g) and dichloromethane (18 ml) were further added to the organic layer, and the mixture was stirred for 30 minutes. Thereafter, activated carbon was filtered off. The residue was washed with dichloromethane (90 ml) and the filtrate and the washing liquid were combined and concentrated under reduced pressure at an internal temperature of 25˚C to a fluid volume of 300 ml. After stirring at normal pressure for 10 minutes, methanol (300 ml) was added and the mixture was concentrated under reduced pressure at an internal temperature of 25˚C to a fluid volume of 300 ml. Methanol (300 ml) was further added and the mixture was concentrated under reduced pressure at an internal temperature of 30˚C to a fluid volume of 300 ml. Methanol (198 ml) was added thereto and the mixture was cooled to 0 to 5˚C and further stirred for one hour. The resulting crystals were separated by filtration, washed with cold methanol (240 ml), and then dried under reduced pressure at 50˚C to obtain tert-butyl N-{2-{4-[(2,4-dioxothiazolidin-5-yl)methyl]phenoxyacetylamino}-5-methoxyphenyl}-N-methylcarbamate (97.09 g, 188.3 mmol) (yield: 89%).

(Example 3)

tert-Butyl N-{2-{4-[(2,4-dioxothiazolidin-5-yl)methyl]phenoxyacetylamino}-5-methoxyphenyl}-N-methylcarbamate

Acetonitrile (400 ml) was added to 4-[(2,4-dioxothiazolidin-5-yl)methyl]phenoxyacetic acid (40.0 g, 142.2 mmol). After cooling to an internal temperature of 7˚C, thionyl chloride (18.4 g, 155.0 mmol) was added. Dimethylformamide (32 ml) was further added and the mixture was stirred at the same temperature to 11.4˚C for three hours.

A solution of tert-butyl N-(2-amino-5-methoxyphenyl)-N-methylcarbamate (38.4 g, 137.9 mmol) and triethylamine (18.7 g, 184.9 mmol) in acetonitrile (240 ml) maintained at 0 to 10˚C was added dropwise thereto over 65 minutes while cooling to maintain the reaction temperature at 0 to 5˚C, and then the mixture was further stirred at the same temperature for two hours. Next, water (320 ml) was added over 15 minutes and the mixture was stirred at an internal

temperature of 0 to 5˚C for 2.5 hours. Thereafter, the precipitated crystals were separated by filtration. The resulting crystals were washed with a 2:1 solution of acetonitrile and water (160 ml) and dried under reduced pressure at 50˚C for 19 hours to obtain crystals of tert-butyl N-{2-{4-[(2,4-dioxothiazolidin-5-yl)methyl]phenoxyacetylamino}-5-methoxyphenyl}-N-methylcarbamate (63.6 g, 123.4 mmol) (yield: 89%).

(Example 4)

tert-Butyl N-{2-{4-[(2,4-dioxothiazolidin-5-yl)methyl]phenoxyacetylamino}-5-methoxyphenyl}-N-methylcarbamate

(4-1)

(The same lots of 4-[(2,4-dioxothiazolidin-5-yl)methyl]phenoxyacetic acid and tert-butyl N-(2-amino-5-methoxyphenyl)-N-methylcarbamate as used in Example 3 were used in this example, respectively).

Acetonitrile (400 ml) was added to 4-[(2,4-dioxothiazolidin-5-yl)methyl]phenoxyacetic acid (40.0 g, 142.2 mmol). After cooling to an internal temperature of 8˚C, thionyl chloride (18.4 g, 155.0 mmol) was added. Dimethylformamide (32 ml) was further added and the mixture was stirred at the same temperature to 12˚C for three hours. A solution of tert-butyl N-(2-amino-5-methoxyphenyl)-N-methylcarbamate (38.4 g, 137.9 mmol) and triethylamine (18.7 g, 184.9 mmol) in acetonitrile (240 ml) maintained at 0 to 10˚C was added dropwise thereto over 65 minutes while cooling to maintain the reaction temperature at 0 to 3˚C, and then the mixture was further stirred at the same temperature for 3.5 hours. Next, water (320 ml) was added over 27 minutes and the mixture was stirred at 0 to 5˚C for 2.5 hours. Thereafter, the precipitated crystals were separated by filtration. The resulting crystals were washed with a 2:1 solution of acetonitrile and water (160 ml) and then dried under reduced pressure at 50˚C for 15 hours to obtain crystals of tert-butyl N-{2-{4-[(2,4-dioxothiazolidin-5-yl)methyl]phenoxyacetylamino}-5-methoxyphenyl}-N-methylcarbamate (67.6 g, 131.0 mmol) (yield: 92%).

(4-2)

A suspension of the crystals of tert-butyl N-{2-{4-[(2,4-dioxothiazolidin-5-yl)methyl]phenoxyacetylamino}-5-methoxyphenyl}-N-methylcarbamate obtained in (4-1) (56.1 g, 108.6 mmol) in methanol (1680 ml) was heated with stirring (the crystals were completely dissolved when the internal temperature reached 65.5˚C). The reaction solution was cooled to 0 to 5˚C over two hours and further stirred at the same temperature for 95 minutes, and then the precipitated crystals were separated by filtration. The resulting crystals were washed with methanol (224 ml) and then dried under reduced pressure at 50˚C for 15 hours to obtain purified crystals of tert-butyl N-{2-{4-[(2,4-dioxothiazolidin-5-yl)methyl]phenoxyacetylamino}-5-methoxyphenyl}-N-methylcarbamate (51.6 g, 100.0 mmol) (yield: 92%, total yield: 85%).

(Example 5)

{5-4-[(6-Methoxy-1-methyl-1H-benzimidazol-2-yl)methoxy]benzyl}thiazolidine-2,4-dione hydrochloride

(5-1)

Thionyl chloride (28.15 kg, 236.6 mol) and dimethylformamide (6.1 L) were poured into a suspension of 4-[(2,4-dioxothiazolidin-5-yl)methyl]phenoxyacetic acid (61.0 kg, 216.9 mol) in dichloromethane (398 L), and the mixture was refluxed for six hours. After cooling the resulting solution to 0 to 5˚C, a solution of tert-butyl N-(2-amino-5-methoxyphenyl)-N-methylcarbamate (54.72 kg, 216.9 mol) and triethylamine (26.35 kg, 260.4 mol) in dichloromethane (562 L) was added dropwise over one hour while maintaining the internal temperature at 5˚C or less, and the mixture was stirred at 0 to 5˚C for 15 minutes. Water (488 L) was poured in with stirring and sodium bicarbonate (24.4 kg) was added (the internal temperature was raised to about 20˚C). Then, dichloromethane (305 L) was poured in and the mixture was stirred for 20 minutes while cooling to 0 to 3˚C. Water (488 L) was poured in, the mixture was stirred at 10 to 20˚C for five minutes and allowed to stand for 30 minutes, and the aqueous layer was discarded. Water (488 L) and then 38% hydrochloric acid (23.8 kg) were poured in, the mixture was stirred for five minutes and then allowed to stand for 10 minutes, and the aqueous layer was discarded. Water (488 L) was poured in, the mixture was stirred for five minutes and then allowed to stand for 12 hours, and the aqueous layer was discarded. A suspension of activated carbon (1.83 kg) in dichloromethane (18 L) was added thereto. After stirring for 30 minutes, activated carbon was separated by filtration. Activated carbon was washed with dichloromethane (92 L) and the filtrate and the washing liquid were combined and concentrated under reduced pressure at an internal temperature of 20 to 30˚C to about 300 L. Methanol (305 L) was poured in and the mixture was concentrated under reduced pressure at an internal temperature of 20 to 30˚C to

about 300 L. Methanol (305 L) was further poured in and the mixture was concentrated under reduced pressure at an internal temperature of 20 to 30˚C to about 300 L. Methanol (201 L) was poured in and the mixture was stirred at 5˚C for one hour. Then, the resulting crystals were separated by filtration, washed with methanol (244 L), and then dried under reduced pressure at 50˚C to obtain tert-butyl N-{2-{4-[(2,4-dioxothiazolidin-5-yl)methyl]phenoxyacetylamino}-5-methoxyphenyl}-N-methylcarbamate (103.9 kg, 201.5 mol) (yield: 93%).

(5-2)

tert-Butyl N-{2-{4-[(2,4-dioxothiazolidin-5-yl)methyl]phenoxyacetylamino}-5-methoxyphenyl}-N-methylcarbamate obtained in (5-1) (97.0 kg, 188.1 mol) was suspended in a solution made of methanol (2803 L), water (43 L) and 38% hydrochloric acid (72.8 kg), and the suspension was refluxed with stirring for five hours. The reaction solution was cooled to 0 to 5˚C, and then stirred for one hour and allowed to stand at the same temperature for 12 hours. The resulting crystals were separated by filtration, washed with methanol (291 L), and then dried under reduced pressure at 50˚C to obtain {5-4-[(6-methoxy-1-methyl-1H-benzimidazol-2-yl)methoxy]benzyl}thiazolidine-2,4-dione hydrochloride (74.9 kg, 172.5 mol) (yield: 92%, overall yield for Example 5: 86%).

Peak patterns at a relative intensity of 9 or more in powder X-ray diffraction (Cu Kα, λ = 1.54 Å) are shown in Table 1.

(Table 1)

| 2θ | d | Relative intensity | 2θ | d | Relative intensity |
|---|---|---|---|---|---|
| 6.18 | 14.29 | 30 | 25.04 | 3.55 | 9 |
| 12.42 | 7.12 | 18 | 25.36 | 3.51 | 10 |
| 16.58 | 5.34 | 13 | 28.32 | 3.15 | 13 |
| 17.82 | 4.97 | 12 | 31.44 | 2.84 | 13 |
| 18.70 | 4.74 | 100 | 32.40 | 2.76 | 17 |
| 22.50 | 3.95 | 22 | 35.56 | 2.52 | 10 |
| 23.08 | 3.85 | 17 | 37.96 | 2.37 | 9 |
| 23.72 | 3.75 | 19 | | | |

(Example 6)

{5-4-[(6-Methoxy-1-methyl-1H-benzimidazol-2-yl)methoxy]benzyl}thiazolidine-2,4-dione hydrochloride

(6-1)

Acetonitrile (140.9 kg) was added to 4-[(2,4-dioxothiazolidin-5-yl)methyl]phenoxyacetic acid (18.0 kg, 64.0 mol). After cooling to an internal temperature of 8˚C, thionyl chloride (8.3 kg, 69.8 mol) was added. Dimethylformamide (14.4 L) was further added and the mixture was stirred at the same temperature to 15˚C for 3.5 hours. A solution of tert-butyl N-(2-amino-5-methoxyphenyl)-N-methylcarbamate (15.7 kg, 62.2 mol) and triethylamine (8.4 kg, 83.0 mol) in acetonitrile (84.6 kg) maintained at 0 to 10˚C was added dropwise thereto over one hour while cooling to maintain the reaction temperature at 0 to 5˚C, and then the mixture was further stirred at the same temperature for two hours. Next, water (144 L) was added over 22 minutes, and the mixture was stirred for 30 minutes while maintaining the internal temperature at 0 to 6˚C and then allowed to stand for 12 hours. The resulting crystals were separated by filtration and then washed with a 2:1 solution of water (54 L) to obtain wet crystals of tert-butyl N-{2-{4-[(2,4-dioxothiazolidin-5-yl) methyl]phenoxyacetylamino}-5-methoxyphenyl}-N-methylcarbamate.

(6-2)

A suspension of the wet crystals of tert-butyl N-{2-{4-[(2,4-dioxothiazolidin-5-yl)methyl]phenoxyacetylamino}-5-methoxyphenyl}-N-methylcarbamate obtained in (6-1) in methanol (450 L) was refluxed with stirring for 25 minutes. The suspension was cooled to 0 to 5˚C and stirred at the same temperature for one hour, and then the precipitated crystals were separated by filtration. The resulting crystals were washed with methanol (54 L) to obtain wet purified

crystals of tert-butyl N-{2-{4-[(2,4-dioxothiazolidin-5-yl)methyl]phenoxyacetylamino}-5-methoxyphenyl}-N-methylcarbamate.

(6-3)

The wet purified crystals of tert-butyl N-{2-{4-[(2,4-dioxothiazolidin-5-yl)methyl]phenoxyacetylamino}-5-methoxyphenyl}-N-methylcarbamate obtained in (6-2) were suspended in methanol (1080 L), and then the suspension was refluxed to prepare a solution. The solution was cooled to 50˚C and filtered over 45 minutes. The residue was washed with methanol (37 L) at 50˚C. The filtrate and the washing liquid were combined, 38% hydrochloric acid (20.9 kg) and then water (11.2 L) were poured in at 48˚C, and the mixture was stirred at the same temperature for six hours. The reaction solution was cooled to 0 to 5˚C, and then stirred at the same temperature for 30 minutes and allowed to stand for 12 hours. The resulting crystals were separated by filtration, washed with methanol (91 L), and then dried under reduced pressure at 50˚C to obtain {5-4-[(6-methoxy-1-methyl-1H-benzimidazol-2-yl)methoxy]benzyl}thiazolidine-2,4-dione hydrochloride (19.5 kg, 44.9 mol) (overall yield for Example 6: 70%).

(Example 7)

Test for quality and stability over time of tert-butyl N-{2-{4-[(2,4-dioxothiazolidin-5-yl)methyl]phenoxyacetylamino}-5-methoxyphenyl}-N-methylcarbamate (compound ($III^1$))

Quality of four samples (the crystals obtained in Example 3, the crystals obtained in Example 3 and stored at 40˚C for 40 days, the purified crystals obtained in Example 4 and the purified crystals obtained in Example 4 and stored at 40˚C for 42 days) was analyzed. Quality was analyzed by the following method.

A solution of about 10 mg/L of the compound ($III^1$) in acetonitrile-water (4:1) was prepared.

About 20 μL of the sample solution was analyzed by high-performance liquid chromatography under the following conditions. (The test substance showed a retention time of about 12 minutes.)
Column: L-column ODS 4.6 × 250 mm (Chemicals Evaluation and Research Institute, Japan)
Mobile phase: 0.02 M ammonium acetate solution/acetonitrile mixture (55:45)
Flow rate: 1 ml/min
Detection wavelength: 220 nm
Measurement temperature: 40˚C
Peak areas were determined from chromatograms, respectively, to provide a content of each component (%) = 100 × (peak area of each component)/(total peak area of all components). The results are shown in Table 2.

(Table 2)

| Relative retention time for test substance | Crystals of Example 3 | | Purified crystals of Example 4 | |
|---|---|---|---|---|
| | Immediately after drying (%) | 40˚C after 40 days (%) | Immediately after drying (%) | 40˚C after 42 days (%) |
| 0.21 | 0.60 | 0.63 | 0.19 | 0.26 |
| 0.43 | 0.39 | 0.32 | 0.07 | 0.07 |
| 0.46 | 0.16 | 0.48 | 0.08 | 0.09 |
| 1.00 (test substance) | 98.26 | 97.96 | 99.37 | 99.35 |
| 3.01 | 0.05 | 0.05 | 0.04 | 0.03 |
| Total of other components | 0.54 | 0.56 | 0.25 | 0.20 |
| Total of impurities | 1.74 | 2.04 | 0.63 | 0.65 |

The test substances were each produced by different processes as described in the above examples. The crystals of Example 4 were produced by a synthesis method according to the following scheme (wherein the symbols in the scheme are defined as described above). On the other hand, the crystals of Example 3 were produced in the following scheme without carrying out purification of Step 3.

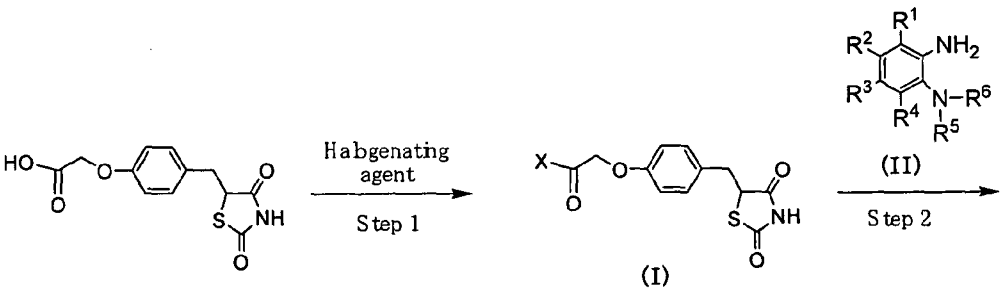


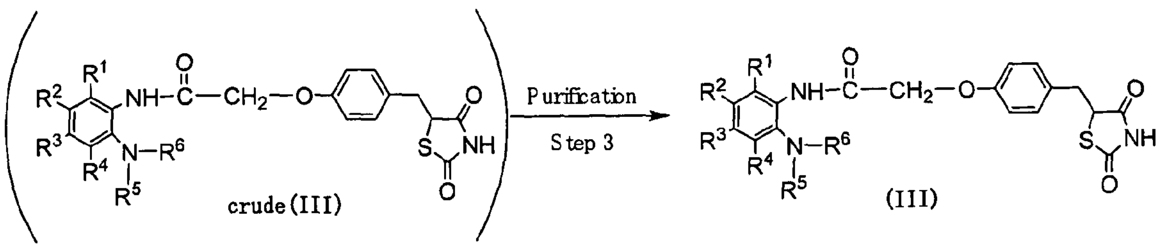


As is clear from Table 2, the amount of impurities is significantly reduced by carrying out the purification operation of Step 3. Table 2 further shows that the crystals having a high purity obtained by the purification operation of Step 3 have highly excellent stability over time, and thus the crystals have properties particularly suitable as a pharmaceutical intermediate.

(Example 8)

Impurity analysis of {5-4-[(6-methoxy-1-methyl-1H-benzimidazol-2-yl)methoxy]benzyl}thiazolidine-2,4-dione hydrochloride (hereinafter compound ($A^1$)) (8-1) Qualitative analysis of impurities in crystals of compound ($A^1$) obtained in Examples 5 and 6

About 0.02 g of the compound ($A^1$) to be analyzed was weighed and dissolved in a 0.5% phosphoric acid solution-acetonitrile (65:35) to prepare 100 ml of a sample solution.

(Test substance 1: compound of Example 5, test substance 2: compound of Example 6)

The sample solution was accurately diluted to 100- fold with a 0.5% phosphoric acid solution-acetonitrile (65: 35) to prepare a standard solution.

Accurately 10 μL each of the sample solution and the standard solution was analyzed by high-performance liquid chromatography under the following conditions. (The test substance showed a retention time of about 10 minutes.)
Column: L-column ODS 4.6 × 150 mm (Chemicals Evaluation and Research Institute, Japan)
Mobile phase: 0.01 M sodium acetate buffer (pH = 5)/acetonitrile mixture (13:7)
Flow rate: 1 ml/min
Detection wavelength: 225 nm

Peak areas were determined from chromatograms, respectively, to provide a content of each component (%)

= [(peak area of each component in sample solution)/(peak area of test substance in standard solution)].

(8-2) Quantitative analysis of crystals of compound ($A^1$) obtained in Examples 5 and 6

Sample dissolving liquid: Acetonitrile-0.5% phosphoric acid solution (35:75)
Internal standard solution: 200 ml of solution of about 1 ml of methyl salicylate in sample dissolving liquid
A standard solution was prepared as follows.
About 0.04 g of a standard product of the compound ($A^1$) was accurately weighed in a 200 ml volumetric flask. About 160 ml of the sample dissolving liquid was added to dissolve the compound. Then, 10 ml of the internal standard solution and the sample solution were added to prepare 200 ml of a liquid. Accurately 10 ml of the liquid was put in a 25 ml volumetric flask and then the sample dissolving liquid was added to prepare 25 ml of the standard solution.
A measurement sample solution was prepared as follows.
About 0.04 g of the sample was weighed in a 200 ml volumetric flask. About 160 ml of the sample dissolving liquid was added to dissolve the sample. Then, 10 ml of the internal standard solution and the sample solution were added to prepare 200 ml of a liquid. Accurately 10 ml of the liquid was put in a 25 ml volumetric flask and then the sample dissolving liquid was added to prepare 25 ml of the measurement sample solution.
The HPLC conditions are as follows.
Detection wavelength: 290 nm
Column: L-column ODS 4.6 x 150 mm (Chemicals Evaluation and Research Institute, Japan)
Mobile phase: 0.01 M sodium acetate buffer (pH = 5)/acetonitrile mixture (13:7)
Flow rate: 1 ml/min (controlled to make the retention time of the internal standard about 17 minutes)
Column temperature: 40˚C
Peak areas were determined from chromatograms, respectively, to calculate the content of the compound ($A^1$) according to the following formula.

$$\text{Content of compound } (A^1)\ (\%) = W_1 \times F_1 \times (Q_T/Q_S) \times [1/\{W_2 \times (100 - F_2)/100\}] \times 100$$

$W_1$: Weighed amount of standard product of compound ($A^1$) (g)
$W_2$: Weighed amount of sample (g)
$F_1$: Purity coefficient of standard product of compound ($A^1$)
$F_2$: Moisture in sample measured by Karl-Fischer moisture meter
$Q_T$: Peak area ratio of sample to internal standard substance
$Q_S$: Peak area ratio of standard product of compound ($A^1$) to internal standard substance
The results of the above analyses of Example 8 are shown in Tables 3 and 4.

(Table 3)

| | Impurity content (%) | |
|---|---|---|
| Relative retention time for test substance | Crystals of Example 5 | Crystals of Example 6 |
| 0.27 | <0.01 | <0.01 |
| 0.29 | 0.01 | <0.01 |
| 0.88 | 0.01 | 0.02 |
| 1.21 | 0.04 | <0.01 |
| 2.81 | 0.01 | <0.01 |
| 3.38 | <0.01 | <0.01 |
| Total of other peaks | 0.16 | 0.07 |
| Total of impurities | 0.23 | 0.09 |

(Table 4)

| | Crystals of Example 5 | Crystals of Example 6 | Comparative Compound*) |
|---|---|---|---|
| Quantitative analysis value (%) | 99.2 | 99.4 | 76*) |

*) Quality of the compound ($A^1$) produced by the process described in WO 00/71540 (comparative compound) was quantitatively analyzed by the method of the reference example to find that the quantitative value was 76%.

The test substances were each produced by different processes as described in the above examples. The crystals of Example 6 were produced by a synthesis method according to the following scheme (wherein the symbols in the scheme are defined as described above). On the other hand, the crystals of Example 5 were purified without carrying out purification of Step 3 in the following scheme. In the process described in WO 00/71540, the compound ($A^1$) is produced in a route not using an intermediate acid halide (I) and without carrying out purification of Step 3.

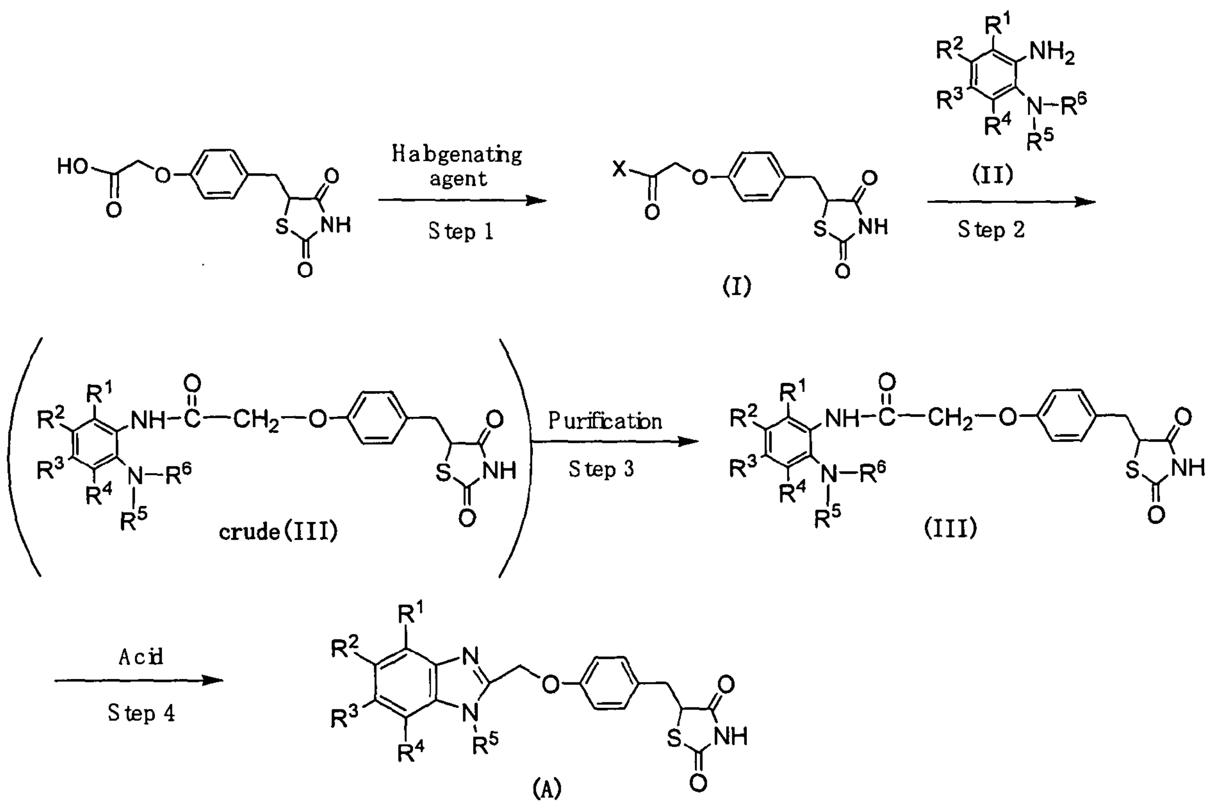


Table 3 shows that the amount of impurities is significantly reduced by carrying out the purification operation of Step 3, and thus the purification method is particularly excellent for removing impurities. Further, it is found that the purification operation of Step 3 is an important operation for producing a high-quality compound (A), because the effect of removing impurities in Step 3 is greatly reflected even in the purity of the final product.

As is clear from the results in Table 4, the process for producing a compound (A) according to the present invention is an industrial production process using a simpler operation but can provide crystals with a higher purity more efficiently, as compared with the known production process described in WO 00/71540 in which crystals are produced with a quantitative value of 76%.

(Reference Example 1)

Quantitative analysis method of {5-4-[(6-methoxy-1-methyl-1H-benzimidazol-2-yl)methoxy]benzyl}thiazolidine-2,4-dione hydrochloride

Column: L-column ODS 4.6 x 150 mm (Chemicals Evaluation and Research Institute, Japan)
Mobile phase: 0.01 M sodium acetate buffer (pH = 5)/acetonitrile mixture (13:7)
Flow rate: 1 ml/min
Detection wavelength: 290 nm
Column temperature: 40˚C
{5-4-[(6-Methoxy-1-methyl-1H-benzimidazol-2-yl)methoxy]benzyl}thiazolidine-2,4-dione hydrochloride was eluted in about 10 minutes under such conditions, and quantitative calculation was carried out from the resulting chromatogram.

(Formulation Example)

(Formulation Example 1)

Tablets

Tablets were obtained by the following method using ingredients whose types and amounts are shown in Table 5. The dose of the active ingredient and the content and type of each additive are not limited to those in Table 5.

An excipient (lactose) and a disintegrant (croscarmellose sodium (Ac-Di-Sol)) were mixed with the crystals of Example 5 in a high-speed stirring granulator. The resulting mixture was kneaded with a binder (hydroxypropylcellulose) solution to obtain a granulated product. The resulting granulated product was dried in a fluid bed dryer, and the dried granulated product was forced to pass through a screen using a crushing granulator and mixed with a lubricant (magnesium stearate) in a V-type mixer. The resulting mixture was formed using a pestle having a diameter of 7 mm, and a coating (Opadry WHITE YS-1-18202A) solution with a pigment (yellow iron sesquioxide) dispersed was sprayed in a coater to obtain desired tablets.

(Table 5)

| Ingredient | Amount per tablet (mg) |
|---|---|
| Crystals of Example 5 | 10.9 |
| Lactose | 94.4 |
| Ac-Di-Sol | 19.5 |
| Hydroxypropylcellulose | 3.9 |
| Magnesium stearate | 1.3 |
| Opadry White YS-1-18202A | 5.972 |
| Yellow iron sesquioxide | 0.028 |
| Total | 136.0 |

## Claims

**1.** A process for producing a compound represented by the following general formula (III):

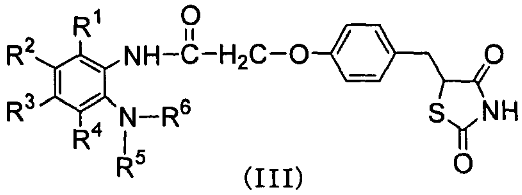

(III)

wherein $R^1$, $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, $R^5$ represents a C1-C6 alkyl group, and $R^6$ represents a protecting group for an amino group, **characterized in that** the process comprises reacting an acid halide compound represented by the following general formula (I):

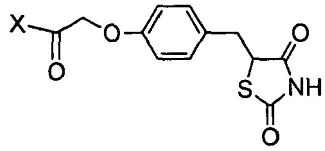

(I)

wherein X represents a halogen atom, with a phenylenediamine compound represented by the following general formula (II):

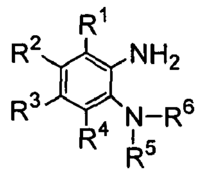

(II)

wherein $R^1$, $R^2$, $R^3$, $R^4$, $R^5$ and $R^6$ are as defined above.

2. The production process according to claim 1, wherein $R^3$ is a C1-C6 alkoxy group.

3. A process for producing tert-butyl N-{2-{4-[(2,4-dioxothiazolidin-5-yl)methyl]phenoxyacetylamino}-5-methoxyphenyl}-N-methylcarbamate or a salt thereof, **characterized in that** the process comprises reacting 4-[(2,4-dioxothiazolidin-5-yl)methyl]phenoxyacetyl chloride with tert-butyl N-(2-amino-5-methoxyphenyl)-N-methylcarbamate.

4. A process for purifying a compound represented by the following general formula (III):

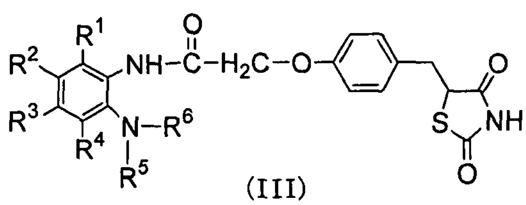

(III)

wherein $R^1$, $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, $R^5$ represents a C1-C6 alkyl group, and $R^6$ represents a protecting group for an amino group, **characterized in that** the process comprises refluxing a suspension or solution of crude crystals of the compound in an organic solvent (wherein the organic solvent is selected from alcohols, ethers, nitriles and mixed solvents thereof).

5. The purification process according to claim 4, wherein the organic solvent is an alcoholic solvent.

6. The purification process according to claim 4 or 5, wherein the compound represented by the general formula (III) is tert-butyl N-{2-{4-[(2,4-dioxothiazolidin-5-yl)methyl]phenoxyacetylamino}-5-methoxyphenyl}-N-methylcarbamate or a salt thereof.

**7.** A process for producing a thiazolidinedione compound represented by the following general formula (A):

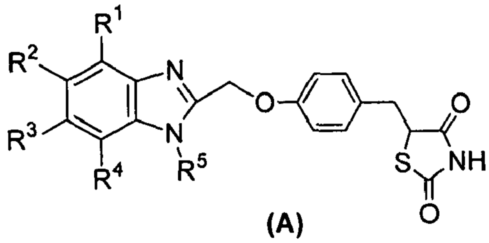

**(A)**

or a pharmacologically acceptable salt thereof,
wherein $R^1$, $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, and $R^5$ represents a C1-C6 alkyl group,
**characterized in that** the process comprises refluxing a suspension or solution of a compound represented by the following general formula (III):

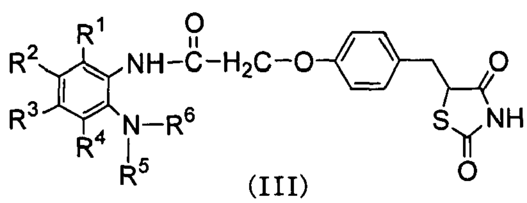

(III)

wherein $R^1$, $R^2$ , $R^3$ , $R^4$ and $R^5$ are as defined above, and $R^6$ represents a protecting group for an amino group, in an organic solvent (wherein the organic solvent is selected from alcohols, ethers, nitriles and mixed solvents thereof) so that the final product has an impurity content of 2% or less.

**8.** A process for producing a thiazolidinedione compound represented by the following general formula (A):

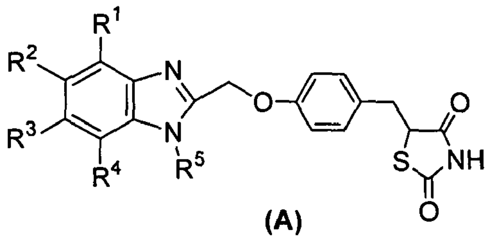

**(A)**

or a pharmacologically acceptable salt thereof,
wherein $R^1$, $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, and $R^5$ represents a C1-C6 alkyl group,
**characterized in that** the process comprises reacting a compound represented by the following general formula (I):

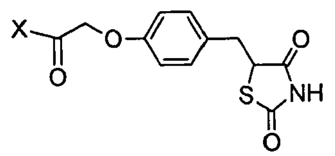

(I)

wherein X represents a halogen atom, with a compound represented by the following general formula (II):

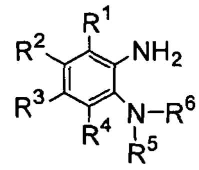

(II)

wherein $R^1$, $R^2$, $R^3$, $R^4$ and $R^5$ are as defined above, and $R^6$ represents a protecting group for an amino group; and treating with an acid the resulting compound represented by the following general formula (III):

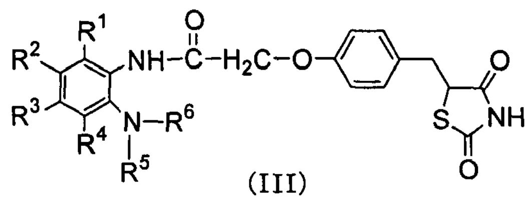

(III)

wherein $R^1$, $R^2$, $R^3$, $R^4$, $R^5$ and $R^6$ are as defined above.

**9.** A process for producing a thiazolidinedione compound represented by the following general formula (A):

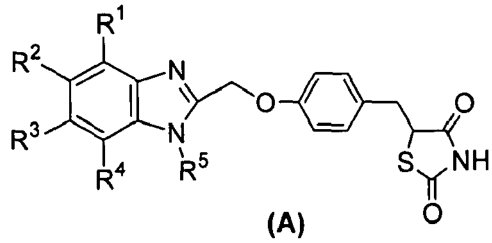

**(A)**

or a pharmacologically acceptable salt thereof,
wherein $R^1$, $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, and $R^5$ represents a C1-C6 alkyl group,
**characterized in that** the process comprises reacting 4-[(2,4-dioxothiazolidin-5-yl)methyl]phenoxyacetic acid with a halogenating agent; reacting the resulting compound represented by the following general formula (I) :

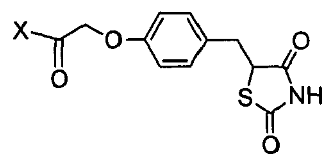

(I)

or salt thereof,
wherein X represents a halogen atom, with a compound represented by the following general formula (II):

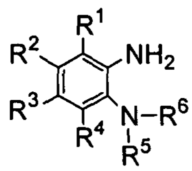

(II)

wherein $R^1$, $R^2$, $R^3$, $R^4$ and $R^5$ are as defined above, and $R^6$ represents a protecting group for an amino group, to obtain crude crystals of a compound represented by the following general formula (III):

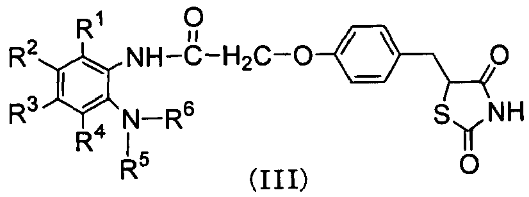

(III)

wherein $R^1$ $R^2$, $R^3$, $R^4$, $R^5$ and $R^6$ are as defined above; refluxing a suspension or solution of the crude crystals in an organic solvent (wherein the organic solvent is selected from alcohols, ethers, nitriles and mixed solvents thereof) to purify the compound; and subsequently cyclizing the compound into an imidazole ring by treatment with an acid.

**10.** A thiazolidinedione compound represented by the following general formula (A):

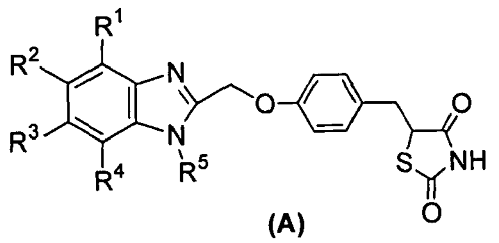

**(A)**

or a pharmacologically acceptable salt thereof having a purity of 98% or more,
wherein $R^1$, $R^2$, $R^3$ and $R^4$ are the same or different and each represents a hydrogen atom, a hydroxyl group, a C1-C6 alkyl group, a C1-C6 alkoxy group, a benzyloxy group, an acetoxy group, a trifluoromethyl group or a halogen atom, and $R^5$ represents a C1-C6 alkyl group.

**11.** A thiazolidinedione compound represented by the general formula (A):

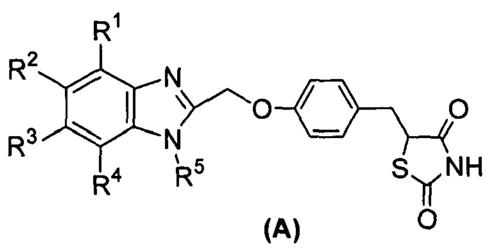

**(A)**

or a pharmacologically acceptable salt thereof,
wherein $R^1$, $R^2$, $R^3$ and $R^4$ are the same or different and each represents a hydrogen atom, a hydroxyl group, a

C1-C6 alkyl group, a C1-C6 alkoxy group, a benzyloxy group, an acetoxy group, a trifluoromethyl group or a halogen atom, and $R^5$ represents a C1-C6 alkyl group,
which is obtained by reacting 4-[(2,4-dioxothiazolidin-5-yl)methyl]phenoxyacetic acid with a halogenating agent; reacting the resulting compound represented by the following general formula (I):

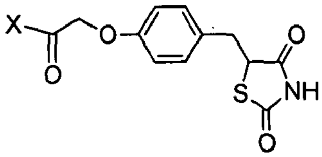

(I)

or salt thereof,
wherein X represents a halogen atom, with a compound represented by the following general formula (II):

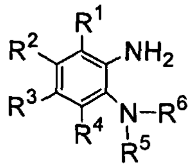

(II)

wherein $R^1$, $R^2$, $R^3$, $R^4$ and $R^5$ are as defined above, and $R^6$ represents a protecting group for an amino group, to obtain a compound represented by the following general formula (III):

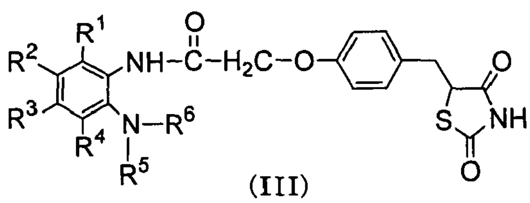

(III)

or a salt thereof,
wherein $R^1$, $R^2$, $R^3$, $R^4$, $R^5$ and $R^6$ are as defined above; refluxing a suspension or solution of the product in an organic solvent (wherein the organic solvent is selected from alcohols, ethers, nitriles and mixed solvents thereof) to purify the product; and subsequently cyclizing the product into an imidazole ring by treatment with an acid.

12. A pharmaceutical composition comprising the compound or pharmacologically acceptable salt thereof according to claim 10 or 11.

13. A pharmaceutical composition comprising the compound or pharmacologically acceptable salt thereof according to claim 10 or 11 for prevention or treatment of diabetes mellitus, hyperglycemia, impaired glucose tolerance, hypertension, hyperlipidemia, diabetic complications, gestational diabetes mellitus, polycystic ovary syndrome or atherosclerosis.

14. A pharmaceutical composition comprising the compound or pharmacologically acceptable salt thereof according to claim 10 or 11 for prevention or treatment of diabetes mellitus.

15. Use of the compound or pharmacologically acceptable salt thereof according to claim 10 or 11 for production of a pharmaceutical composition for prevention or treatment of diabetes mellitus.

16. A compound represented by the following general formula (I):

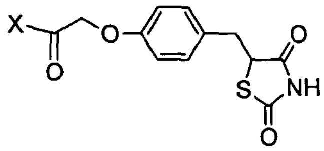

(I)

or a salt thereof,
wherein X represents a halogen atom.

**17.** The compound or salt thereof according to claim 16, wherein X is a chlorine atom.

**18.** A process for producing a compound represented by the following general formula (I):

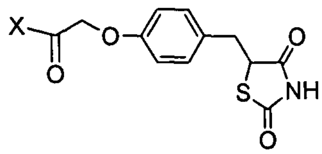

(I)

or a salt thereof,
wherein X represents a halogen atom,
**characterized in that** the process comprises reacting 4-[(2,4-dioxothiazolidin-5-yl)methyl]phenoxyacetic acid with a halogenating agent.

**19.** The production process according to claim 18, wherein X is a chlorine atom.

[Figure 1]

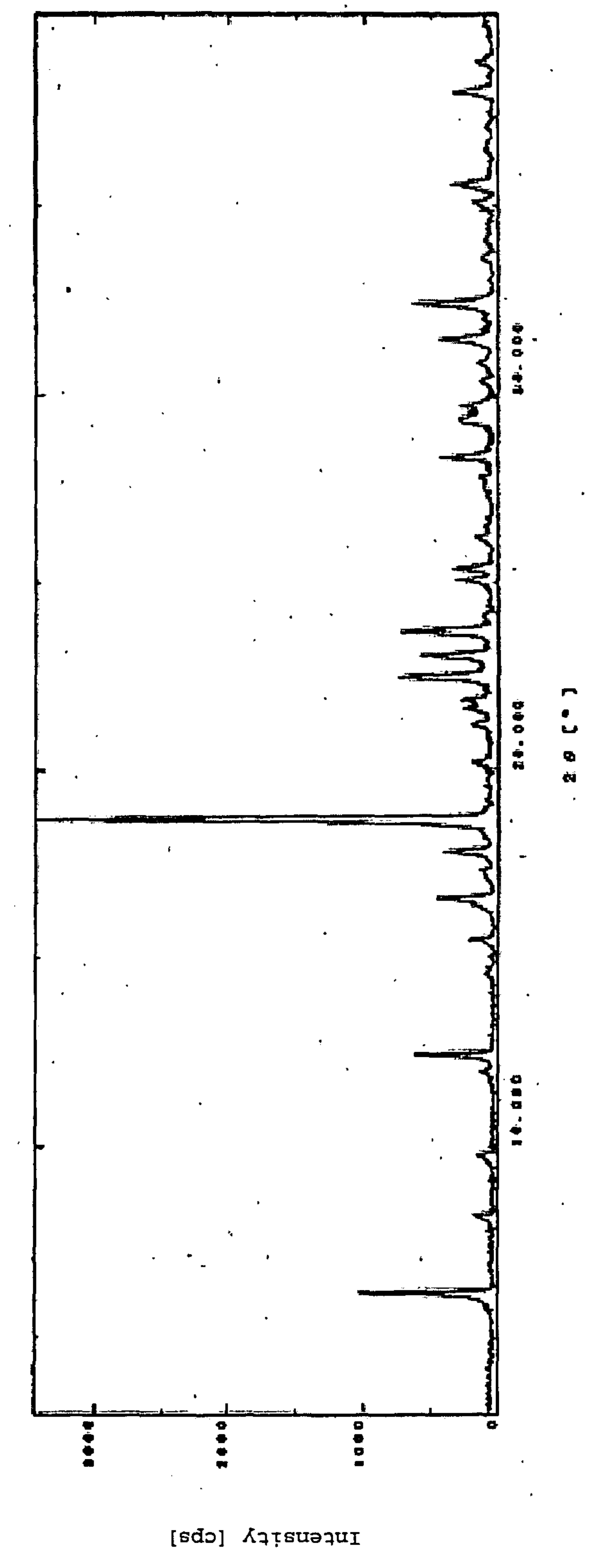

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001072671 A
- JP 9095970 A
- JP 2001039976 A
- WO 0071540 A

### Non-patent literature cited in the description

- *Journal of Chemical Society Perkin Transactions I,* 1992, 973-978
- *Bioorganic & Medicinal Chemistry Letters,* 2004, vol. 14, 235-238